# EUROPÄISCHE PATENTSCHRIFT

(11) **EP 1 429 628 B1**
(45) Veröffentlichungstag und Bekanntmachung des Hinweises auf die Patenterteilung: **27.04.2005**
(21) Anmeldenummer: 02799375.7
(22) Anmeldetag: 25.09.2002
(51) Int. Cl.: A23L 1/30, A23L 2/38, A23L 2/52, A23L 1/304, A23L 1/302

(54) **BASENHÄLTIGE MIKRONÄHRSTOFFMISCHUNG**
MIXTURE OF BASE-CONTAINING MICRONUTRIENT SUBSTANCES
MELANGE DE SUBSTANCES MICRONUTRITIVES RENFERMANT DES BASES

(30) Priorität: 27.09.2001 AT 15342001
(43) Veröffentlichungstag der Anmeldung: 23.06.2004
(73) Patentinhaber: NUTROPIA Ernährungsmedizinische Forschungs GmbH, 5580 Unternberg (AT)
(72) Erfinder: FUCHS, Norbert, A-5571 Mariapfarr (AT); KÖSSLER, Peter, A-5571 Mariapfarr (AT)
(74) Vertreter: Sonn & Partner Patentanwälte
(86) Internationale Anmeldenummer: PCT/AT2002/000279
(87) Internationale Veröffentlichungsnummer: WO 2003/026444

(56) Entgegenhaltungen:
- WO-A-91/11117
- WO-A-96/03059
- US-A- 4 871 550
- US-A- 5 104 677
- US-B1- 6 254 904

## Beschreibung

Die Erfindung betrifft basenhältige Mikronährstoffmischungen.

Die gezielte Sportlerernährung gilt neben einem ausgezeichneten Training und einer entsprechenden mentalen Einstellung als eine der Säulen für die optimale körperliche Leistungsfähigkeit. Gerade die gezielte Sportlerernährung wird jedoch als eine der Voraussetzungen für eine optimale Leistungsfähigkeit immer wieder unterschätzt.

Aufgrund belegter Beziehungen zwischen Defiziten in der Versorgung mit Mikronährstoffen und Einbußen in der Leistungsfähigkeit, kommt dabei der Versorgung des Sportlers mit Mikronährstoffen und der Beurteilung des Stoffwechsels einzelner Mikronährstoffe bei akuter und chronischer körperlicher Mehrbelastung zunehmend Bedeutung zu.

Der weitaus größte Teil des Energiebedarfs wird bei körperlicher Arbeit, besonders bei längerer Arbeitsdauer, durch die aerobe oxidation der Nährstoffe bereitgestellt. Der zweite Weg der Energiebereitstellung, die anaerobe Oxidation, wird dann beschritten, wenn der momentane Energiebedarf nicht durch die aerobe Oxidation gedeckt werden kann. Die Kapazität der Energiebereitstellung durch aerobe Oxidation wird vor allem begrenzt durch:
- das Sauerstoffangebot an die Zielzelle,
- die Kapazität diverser Enzymsysteme, die die einzelnen Reaktionen bei der Oxidation katalysieren,
- die Größe der interzellulären und humoralen Pufferkapazitäten.

Hauptmerkmal der anaeroben Energiebereitstellung (anaerobe Glykoloyse) ist die Bildung von Lactat. Bis zu einer bestimmten Belastungsintensität erfolgt im Körper der Abbau von Lactat schneller als seine Entstehung. Wenn das produzierte Lactat gerade noch vom Körper abgebaut werden kann, spricht man von "anaerober Schwelle". wird allerdings diese Schwelle überschritten, kann das produzierte Lactat nicht mehr schnell genug vom Körper beseitigt werden. Die Folge ist eine signifikante Steigerung der Muskelund Blutlactatwerte. Die sich in der Muskelzelle anhäufende Milchsäure verändert den intrazellulären pH-Wert und schränkt Enzymaktivitäten ein. Obwohl ein Großteil der anfallenden H⁺-Ionen durch Bicarbonat abgepuffert wird und die Milchsäure relativ rasch vor allem an das Blut abgegeben wird, kommt es zur Muskelermüdung, und der Organismus ist gezwungen, seine Tätigkeit zu drosseln oder ganz einzustellen.

In Ruhe beträgt der Lactat-Wert ca. 0,5 - 1 mmol/l Blut. Unter maximalen körperlichen Belastungen kann dieser Wert auf bis 20 - 30 mmol/l ansteigen. An der anaeroben Schwelle (ANS) beträgt der Lactat-Wert ca. 5 mmol/l Blut.

Die Beseitigung des angefallenen Lactats geschieht nach der Belastung zum Teil in der Zelle durch Wiederaufbau zu Glykogen bzw. Weiterverarbeitung mit Energiegewinn im aeroben Stoffwechsel. Aus dem Blut wird Milchsäure durch oxidative Verbrennung über den Herzmuskel und die Aufnahme in Leber, Nieren und unbelastete Muskulatur entfernt. Die Eliminationsrate aus dem Blut beträgt 0,5 mmol/min bei Vorliegen von Lactat-Konzentrationen von > 5 mmol/l. In Zusammenhang mit der Lactatproduktion ist auch die Pufferkapazität von Blut und Muskulatur von Interesse. Das ins Blut übertretende Lactat wird überwiegend durch das Bicarbonat-Puffersystem abgepuffert.

Bislang im Stand der Technik erhältliche iso- oder hypotonische Sportgetränke zielen ausschließlich auf Wasser-, Kohlenhydratoder Elektrolytzufuhr ab. Eine gezielte Beeinflussung der anaeroben Schwellenleistung bzw. von. Pufferkapazitäten zur Verbesserung des aeroben Energiepotentials kann aber mit derartigen Mitteln nicht erreicht werden.

Die US4871550A betrifft eine oral einnehmbare Nährstoffzusammensetzung für Athleten, die Elektrolyte, Vitamine, sowie Calciumgluconat beinhalten kann und zur Verbesserung der Ausdauer und Leistungsfähigkeit von Sportlern verwendet werden soll. Die JP59/220177A offenbart ein Sportlergetränk, enthaltend Natriumhydrogencarbonat.

Die vörliegende Erfindung stellt sich daher zur Aufgabe, das aerobe Energiepotential zu verbessern und eine Zusammensetzung zur Verfügung zu stellen, mit der ein derartig verbessertes aerobes Energiepotential erzielt werden kann.

Diese Aufgabe wird erfindungsgemäß gelöst durch die Verwendung von Lösungen von Salzen organischer Säuren, ausgewählt aus Hydrogencarbonaten oder Carbonaten, zur Beschleunigung des Lactatabbaues.

Es zeigte sich erfindungsgemäß auch, dass Lösungen von Salzen organischer ausgewählt aus Hydrogencarbonaten oder Carbonaten. Säuren zur Erhöhung der anaeroben Schwellenleistung einsetzbar sind. Damit eignen sich solche Lösungen von Salzen organischer Säuren besonders gut, um als Basis für ein Sportlergetränk verwendet zu werden.

Bevorzugte Weise werden die erfindungsgemässen Hydrogencarbonate oder Carbonate mit weiteren salzen anderer organischer Säuren aus einem oder mehreren Salzen von ausgewählten organischen Säuren mit einem C₁-C₆-Grundkörper, insbesondere Citrate, Hydrogencitrate, Acetate, Gluconate oder Tartrate, Citrate, Hydrogencitrate, Salze anderer C₂- bis C₆-Säuren. Dabei können die Salze erfindungsgemäß alleine oder in Kombination von zwei oder mehreren derartigen Salzen verwendet werden.

Bevorzugterweise können dabei Na-, K-, NH₄-, Ca- oder Mg-Salze der organischen Säuren eingesetzt werden.

Die vorliegende Erfindung betrifft gemäß einem weiteren Aspekt eine Wässrige basenhaltige Mikronährstoffmischung enthaltend Salze organischer Säuren, ausgewählt aus Hydrogencarbonaten oder Carbonaten, Vitamine des B-Komplexes, Vitamin C, Eisen, Chrom, Selen, Zink, Mangan und Kupfer, gelöst, wobei die Mikronährstoffmischung in wässeriger Lösung einen osmotischen Druck von 650 kPa oder weniger aufweist. Die Salze organischer Säuren können dabei ausgewählt sein aus einer einzigen Salzspezies oder Mischungen verschiedener Salze. Vorzugsweise enthält die erfindungsgemäße Zusammensetzung ein oder mehrere der oben ausgewiesenen Salze. Diese erfindungsgemäße Zusammensetzung ist eine speziell für die erfindungsgemäßen Verwendungen geeignete Basis, z.B. für ein Sportlergetränk, da damit die Aufgaben der vorliegenden Erfindung besonders vorteilhaft gelöst werden können.

Es zeigte sich, dass überraschenderweise mit einer derartigen erfindungsgemäßen Verwendung bzw. besonders mit der erfindungsgemäßen Präparation das aerobe Energiepotential deutlich verbessert werden kann, indem
- das Sauerstoffangebot an die Zielzellen durch gezieltes körperliches Training gesteigert,
- die Kapazität der Redox-Enzyme durch gezielte Mikronährstoffzufuhr optimiert und
- die interzellulären und humoralen Pufferkapazitäten durch gezielte Zufuhr alkalischer Elektrolyte erhöht wird.

Mit der erfindungsgemäßen Verwendung sind nunmehr alle diese Effekte durch die gezielt gewählten Inhaltsstoffe erzielbar.

Es zeigte sich, dass für die Neutralisation des im Zuge von körperlicher Leistung, etc., anflutenden Lactats im Zuge der anaeroben Energiebereitstellung und der dadurch hervorgerufenen Anhebung der individuellen anaeroben Schwellenleistung (IANS; wird in Watt gemessen) neben Salzen organischer Säuren, insbesondere Hydrogencarbonaten, auch Vitamine und Spurenelemente als Enzymaktivatoren vorteilhaft sind. Die Energiegewinnung über anaerobe Oxidation ist für die Zelle ein Ausweg, vermehrt benötigte Energie auch dann noch zur Verfügung stellen zu können, wenn die Enyzmsysteme der Atmungskette ihre maximale Umsatzrate bzw. Kapazität erreicht haben.

Letztendlich ist nicht alleine das Sauerstoffangebot in den Mitochondrien als limitierender Faktor für die aerobe Energiebereitstellung anzusehen, sondern die erschöpfte Kapazität der Enzymsysteme (hervorgerufen durch Vitamin- und Spurenelementdefizite), die das Pyruvat zur aeroben Energiebereitstellung weiterverarbeiten, die die Zelle dazu zwingt, den erhöhten Energiebedarf durch den Ausweg der anaeroben Oxidation zu decken. Eine erhöhte Lactatproduktion ist die Folge.

Die dem erfindungsgemäßen Präparat beigefügten Vitamine und die Spurenelemente erhöhen die Kapazität von Enzymsystemen und verbessern das zelluläre Sauerstoffangebot. Besondere Bedeutung kommt dabei den Spurenelementen Eisen, Chrom, Selen, Zink, Mangan und Kupfer sowie den Vitaminen des B-Komplexes und Vitamin C zu.

Spurenelemente sind Bestandteile von Enzymen oder anderen Wirkstoffen. Sie greifen somit regulierend in die verschiedensten Bereiche des Stoffwechsels ein. Aus sportmedizinischer Sicht sind Eisen, Zink, Chrom und Selen von besonderer Bedeutung.

Eisen erfüllt im Stoffwechsel vielfältige Funktionen, insbesondere als Bestandteil sauerstoffübertragender Wirkgruppen (Hämoglobin, Myoglobin, Cytochrome). Für Sportler ist eine ausreichende Eisenversorgung von besonderer Bedeutung, da der Sauerstofftransport im Blut vermehrt beansprucht wird und der Organismus eine größere Blutmenge bildet. Ist die Eisenversorgung defizitär, werden ungenügend Erythrozyten gebildet und die Sauerstofftransportfähigkeit des Blutes wird eingeschränkt. Folgen einer ungenügenden Sauerstoffversorgung infolge nicht optimaler Eisenversorgung sind u.a. Abgeschlagenheit, Müdigkeit, Konzentrationsschwäche, frühzeitige Übersäuerung der Muskulatur, Schlaflosigkeit und Kreislaufstörungen. Die Kombination von Eisen und Vitamin C im Präparat erhöht die Eisenresorption (Vitamin C gilt als Resorptionsförderer).

Zink ist essentielles Spurenelement und Bestandteil und Aktivator von ca. 100 Enzymen des Intermediärstoffwechsels. Als Bestandteil der Carboanhydrase unterstützt Zink die Rückresorption säurebindenden Bicarbonats und leistet damit einen wertvollen Beitrag in der Regulation des Säure-Basen-Haushaltes.

Chrom ist essentielles Element des Kohlenhydratstoffwechsels. Die Vitamine des B-Komplexes sind essentielle Cofaktoren im Stoffwechsel von Eiweiß, Fett und Kohlenhydraten.

Während iso- oder hypotonische Getränke gemäß dem Stand der Technik ausschließlich der Rehydratation dienen, kann mit dem erfindungsgemäßen Präparat bzw. durch die erfindungsgemäße Verwendung über den Rehydratationseffekt hinaus ein verzögertes Anfluten des Lactats und somit eine Verbesserung der IANSL erzielt werden. Da Lactat unter körperlicher Belastung den primär leistungslimitierenden Faktor darstellt, bedeutet die Verbesserung der IANSL in der Praxis für den Freizeit- und Leistungssportler, aber auch für sportlich nicht aktive Menschen, eine optimierung der aeroben Leistungsfähigkeit durch Optimierung der Redox-Enzymsysteme (durch Mikronährstoffe) und durch Verbesserung der Pufferkapazitäten mittels alkalischer Elektrolyte.

Demgegenüber befassen sich Rehydratations-Getränke, also iso- und hypotonische Durstlöscher gemäß dem Stand der Technik, hauptsächlich mit dem Effekt von Kohlenhydrat- und Elektrolytzugaben. Das erfindungsgemäße Präparat entspricht jedoch den Forderungen für ein optimales Sportlergetränk im Sinne einer Steigerung der zellulären Enzymaktivität und daraus resultierend in einer besseren Nutzung des aeroben Stoffwechsels, was zu einem verzögerten und verminderten Anfluten von Lactat führt.

Die erfindungsgemäße Mikronährstoffmischung weist in wässeriger Lösung einen osmotischen Druck von 650 kPa oder weniger auf, und liegt damit deutlich unter den 799,5 kPa für eine physiologische Kochsalzlösung bzw. 763,0 kPa für Blut bei 37°C. Die erfindungsgemäße Präparation ist also damit als zumindest leicht hypotonisch anzusehen.

Vorzugsweise weist die erfindungsgemäße Mikronährstoffmischung einen pH in wässeriger Lösung von 7,5 oder höher, noch bevorzugter von 8,0 oder höher, insbesondere 8,5 oder höher, auf. Bevorzugter Weise ist die erfindungsgemäße Mikronährstoffmischung in vivo-basenbildend. Dies lässt sich neben den basischen Hydrogencarbonat- und Carbonat-Elektrolyten auch durch andere Salze von organischen Säuren (z.B. Gluconate, Citrate, Hydrogencitrate,...) erreichen.

Neben den erfindungsgemäß vorgesehenen Inhaltskomponenten können weiters andere Inhaltsstoffe im vorliegenden Präparat vorgesehen werden, insbesondere Vitamine und Spurenelemente (als Enzymaktivatoren) . Vorzugsweise enthält daher die erfindungsgemäße Zusammensetzung weiters Vitamin E, Provitamin A, Molybdän, Magnesium, Chlorid, Natrium, Calcium, Kalium, Phosphat oder Mischungen dieser Substanzen.

Wie erwähnt, unterscheidet sich das erfindungsgemäße Präparat von herkömmlichen isotonischen Getränken die sich am Markt befinden, die hauptsächlich auf eine Wasser-, Elektrolyt- bzw. Kohlenhydratzufuhr ausgerichtet sind. Demgegenüber enthält das erfindungsgemäße Präparat vorzugsweise einen geringen Kohlenhydratanteil von unter 30 %, insbesondere von unter 20 %, bezogen auf das Gesamtgewicht der trockenen Zusammensetzung.

Weiters ist die vorliegende Zusammensetzung im Wesentlichen frei an Fetten, d.h., dass der Fettgehalt unter 1 %, vorzugsweise unter 0,5 %, liegt. Ebenfalls bevorzugt ist eine Zusammensetzung, die im Wesentlichen frei an Proteinen ist und sich beispielsweise durch einen Eiweißgehalt von unter 2 %, insbesondere von unter 1 %, aufweist.

Alternativ dazu kann aber eine Variation der erfindungsgemäßen Zusammensetzung auch hoch ungesättigte Fettsäuren (z.B. aus naturbelassenen Pflanzenölen) umfassen.

Die Vitamine des B-Komplexes sind bevorzugterweise ausgewählt aus Vitamin B1, B2, B6, B12, Biotin, Folsäure, Pantothensäure, Niacin und Mischungen davon.

Die Inhaltsstoffe der vorliegenden Erfindung können vorzugsweise in einer Form bereitgestellt werden, die eine leichte Aufnahme/Resorption durch den Körper ermöglichen. Dabei sind besonders diejenigen Salzformen der erfindungsgemäßen Inhaltsstoffe bevor- . zugt, die sich bislang physiologisch als gut resorbierbar gezeigt haben. Je nach Einzelkomponente kann daher die anionische/kationische Salzform, Esterform, Hydrochlorid- oder Hydratsalze oder ähnliche Derivate jeweils bevorzugt sein.

Gemäß bevorzugter Ausführungsformen der erfindungsgemäßen Zusammensetzung in Bezug auf die relativen Mengenverhältnisse der Einzelkomponenten weist sie unabhängig voneinander Salze organischer Säuren, ausgewählt aus Hydrogencarbonaten oder Carbonaten, in einer Menge, von 0,2 bis 20 %, vorzugsweise von 1 bis 10 %, insbesondere von 2 bis 7 %, Vitamine des B-Komplexes in einer Menge von 0,0001 bis 2 %, vorzugsweise von 0,001 bis 1%, insbesondere von 0,01 bis 0,5 %, Vitamin C in einer Menge von 0,001 bis 5 %, vorzugsweise von 0,01-bis 2 %, insbesondere von 0,1 bis 1 %, Eisen in einer Menge von 0,0001 bis 0,5 %, vorzugsweise von 0,001 bis 0,2 %, insbesondere von 0,01 bis 0,1 %, Chrom in einer Menge von 0,000001 bis 0,01 %, vorzugsweise von 0,00001 bis 0,001 %, insbesondere von 0,0001 bis 0,001 %, Selen in einer Menge von 0,000001 bis 0,01 %, vorzugsweise von 0,00001 bis 0,001 %, insbesondere von 0,0001 bis 0,001 %, Zink in einer Menge von 0,0001 bis 0,5 %, vorzugsweise von 0,001 bis 0,2 %, insbesondere von 0,01 bis 0,1 %, Mangan in einer Menge von 0,00001 bis 0,1 %, vorzugsweise von 0,0001 bis 0,01 %, insbesondere von 0,001 bis 0,01 % und Kupfer in einer Menge von 0,00001 bis 0,1 %, vorzugsweise von 0,0001 bis 0,01 %, insbesondere von 0,001 bis 0,01 %, jeweils bezogen auf das Gesamtgewicht der trockenen Zusammensetzung, auf.

Wie erwähnt, sind die angegebenen Werte für die einzelnen Inhaltsstoffe unabhängig voneinander zu betrachten. Eine bevorzugte Ausführungsform der erfindungsgemäßen Zusammensetzung betrifft aber eine Zusammensetzung, in der alle der oben angegebenen Relativmengen in der angegebenen Prozentverteilung zugegen sind.

Die erfindungsgemäße Zusammensetzung enthält bevorzugterweise weiters Karotten- und andere Gemüseextrakte, Orangen- und andere Fruchtpulver, Zitronensäure und andere organische Säuren, beta-Carotin, Anthocyane und andere Farbstoffe sowie Aspartame und andere Süßungsmittel.

Die erfindungsgemäße Zusammensetzung wird in wässeriger Lösung verabreicht und kann daher in dieser Form leicht als Sportgetränk zur Verfügung gestellt werden.

Die flüssige Form ist für die unmittelbare Anwendung, z.B. als Sportgetränk, bevorzugt.

Bevorzugterweise liegt die erfindungsgemäße Zusammensetzung in Dosisform vor bzw. wird als Dosisform verkauft, vorzugsweise in einer Tagesdosisform oder in einer Einzeldosisform, die z.B. 2 bis 5 mal, insbesondere 3 mal pro Tag konsumiert werden kann. Diese Dosisformen können dabei schon in flüssiger Form vorgesehen werden.

Bevorzugterweise enthält die erfindungsgemäße Zusammensetzung Cyanocobalamin, Natriumselenit, Natriummolybdat, Chrom-III-chlorid-hexahydrat, Riboflavin, Aneurin-HCl, Pyridoxol-HCl, Calciumpantothenat, dl-alpha-Tocopherol, Kupfergluconat, Manganglucönat, Zinkgluconat, Eisengluconat, Natriumascorbat oder Mischungen davon. Diese speziellen Formen der erfindungsgemäßen Komponenten haben sich als besonders gut geeignet für eine effiziente Aufnahme der erfindungsgemäßen Zusammensetzung erwiesen.

Weiters enthält die erfindungsgemäße Zusammensetzung bevorzugterweise Zitronensäure und andere Säuerungsmittel, Orangenfruchtpulver und andere Frucht-Extrakte und -Aromen, Karottenextrakt und andere *Obst*- und *Gemüse-Extrakte*, Calciumglycerophosphat, Magnesiumglycerophosphat, Natriumchlorid, Süßungsmittel, insbesondere *Aspartam und andere* oder Mischungen dieser Komponenten.

die erfindungsgemäße Zusammensetzung wird, wie erwähnt, als wässerige Lösung verabreicht, wobei sie in einer Konzentration von 0,5 bis 200 g, insbesondere 2 bis 70 g, insbesondere von 5 bis 20 g, jeweils Trockengewicht.pro 250 ml Wasser konsumiert wird.

Bevorzugterweise enthält die erfindungsgemäße Zusammensetzung Natrium-, Ammonium-, Kaliumhydrogencarbonat oder Mischungen davon, aber auch Carbonate, Gluconate, Citrate, Mono- und die Hydrogencitrate, Tartrate und Salze anderer organischer Säuren oder Mischungen davon.

Selen wird bevorzugterweise in Selenit- oder Selenat-Form in der vorliegenden Zusammensetzung vorgegeben. Die Metalle werden bevorzugterweise in Gluconat-Form oder in Form anderer gut verträglicher und gut resorbierbarer Form vorgesehen.

Bevorzugterweise enthält die erfindungsgemäße Zusammensetzung Natrium-, Kalium-, Calcium-, Magnesium-Carbonat, -Bicarbonat, Natrium-, Kalium-, Calcium-, Magnesium-Salze von organischen Säuren, insbesondere -Citrat oder -Tartrat, oder Mischungen dieser Komponenten.

Wie erwähnt, kann mit der vorliegenden Erfindung das Sauerstoffangebot an die Zielzelle gesteigert werden, die Kapazität der Redoxenzyme optimiert werden und die interzellulären und humoralen Pufferkapazitäten erhöht werden.

Gemäß einem weiteren Aspekt betrifft die vorliegende Erfindung daher die Verwendung der erfindungsgemäßen Zusammensetzung zur Beschleunigung des Lactatabbaues zur Verbesserung der Ausdauer bzw. Leistungsfähigkeit, zur Erhöhung der anaeroben Schwellenleistung und zur Verhinderung oder Verzögerung von Ermüdungserscheinungen.

Diese Verwendungen sind besonders geeignet, um als Basis für ein leistungssteigerndes und -verlängerndes Getränk herangezogen zu werden. Ein bevorzugtes Einsatzgebiet ist daher auch die Verwendung der erfindungsgemäßen Zusammensetzung im Rahmen von sportlichen Tätigkeiten, insbesondere im Zuge des Trainings und der Regeneration.

Die Erfindung wird an Hand der nachfolgenden Beispiele, auf die sie selbstverständlich nicht eingeschränkt sein soll, näher erläutert.

### Beispiele:

### Methodik:

**Studiendesign:** monozentrisch, placebokontrolliert, doppelblind
**Studienzentrum:** Institut SportMed, Institut für für sportwissenschaftliche Leistungsdiagnostik - Trainingssteuerung-Forschung, Wien
**Teilnehmerkollektiv:** 40 Personen (26 männlich, 14 weiblich im Alter zwischen 20 und 40 Jahren), davon 20 Verum und 20 Placebo
**Studiendauer:** 10 Wochen
**Prüfsubstanz:** Die Studienteilnehmer verzehrten 3 mal täglich eine Zubereitung folgender Zusammensetzung (à ca. 9,3 g, aufgelöst in ¼ 1 Wasser jeweils eine ½ Stunde vor den Mahlzeiten):
**Zutaten:** Säuerungsmittel Zitronensäure, Calciumglycerophosphat, Orangenfruchtpulver, Kaliumhydrogencarbonat, Karottenextrakt, Magnesiumglycerophosphat, Natriumchlorid, Natriumhydrogencarbonat, Natriumascorbat, Beta-Carotin, Eisengluconat, Zinkgluconat, Süßstoff Aspartame, Mangangluconat, Niacin, Kupfergluconat, Vitamin E, Pantothensäure, Vitamin B6, Vitamin B1, Vitamin B2, Chrom-III-chlorid-hexahydrat, Natriummolybdat, Natriumselenit, Folsäure, Biotin, Vitamin B12.

**Tabelle 1**

| Dosis pro Tag (entspricht 27,9 g): | | | |
|---|---|---|---|
| Vitamin C | 150,00 mg | Natrium | 319,50 mg |
| Niacin | 18,00 mg | Chlorid | 272,46 mg |
| Vitamin E | 9,99 mg | Magnesium | 249,99 mg |
| Pantothensäure | 6,00 mg | Eisen | 15,00 mg |
| Vitamin B6 | 2,01 mg | Zink | 15,00 mg |
| beta-Carotin | 1,62 mg | Mangan | 5,01 mg |
| Vitamin B2 | 1,59 mg | Kupfer | 2,01 mg |
| Vitamin B1 | 1,41 mg | Molybdän | 200,00 mcg |
| Folsäure | 200,00 mcg | Chrom | 200,00 mcg |
| Biotin | 50,00 mcg | Selen | 100,00 mcg |
| Vitamin B12 | 1,00 mcg | | |
| Phosphor | 1042,38 mg | Kohlenhydrate | 5,82 g |
| Kalium | 999,99 mg | Eiweiß | 0,15 g |
| calcium | 999,99 mg | Fett | 0,06 g |

Gemäß der Isotonieberechnungen beträgt der osmotische Druck des erfindungsgemäßen Präparates 630,1 kPa vs. 799,5 kPa für eine physiologische Kochsalzlösung vs. 763,0 kPa für Blut bei 37°C. Das erfindungsgemäße Präparat ist damit leicht hypotonisch.

**Placebo:** Gemäß der Isotonieberechnungen beträgt der osmotische Druck des Placebos 621,5 kPa vs. 799,5 kPa für eine physiologische Kochsalzlösung vs. 763,0 kPa für Blut bei 37°C. Das eingesetzte Placebo ist damit ebenso leicht hypotonisch. Die Placebo-Probe bestand aus einer Mischung aus Fructose, Glucose, Zitronensäure, Orangenaroma, Karottenextrakt und Süßstoff Äspartame.

**Tabelle 2:**

| Form, Konzentration und Zusammensetzung der Mikronährstoffe | | | |
|---|---|---|---|
| Nährstoffe pro Einzeldosis | | Zusammensetzung pro Einzeldosis | |
| Vitamin B12 | 0,0003 mg | Cyanpcobalamin | 0,00033 mg |
| Biotin | 0,0167 mg | Biotin | 0,01667 mg |
| Folsäure | 0,0667 mg | Folsäure | 0,06667 mg |
| Selen | 0,0333 mg | Natriumselenit | 0,11099 mg |
| Molybdän | 0,0667 mg | Natriummolybdat | 0,16801 mg |
| Chrom | 0,0667 mg | Chrom-III-chlorid-hexahydrat | 0,341 mg |
| Vitamin B2 | 0,53 mg | Riboflavin | 0,530 mg |
| Vitamin B1 | 0,47 mg | Aneurin-HCl | 0,600 mg |
| Vitamin B6 | 0,67 mg | Pyridoxol-HCl | 0,790 mg |
| Pantothensäure | 2,00 mg | Calciumpantothenat | 2,16 mg |
| Vitamin E | 3,33 mg | Dl-alpha-Tocopherol | 3,33 mg |
| Kupfer | 0,6700 mg | Kupfergluconat | 4,87 mg |
| Niacin | 6,00 mg | Nikotinsäureamid | 6,00 mg |
| Mangan | 1,67 mg | Mangangluconat | 13,54 mg |
| Zink | 5,00 mg | Zinkgluconat | 34,85 mg |
| Eisen | 5,00 mg | Eisengluconat | 38,40 mg |
| beta-Carotin | 0,54 mg | beta-Carotin 1 % | 54,00 mg |
| Vitamin C | 50,00 mg | Natriumascorbat | 56,00 mg |

### Begleitende Maßnahmen:

Alle 40 Teilnehmer absolvierten über den 10-wöchigen Prüfzeitraum 3 mal wöchentlich ein 1-stündiges Trainingsprogramm unter Kontrolle eines diplomierten Trainers.

### Untersuchungsparameter:

Gesamtcholesterin, HDL-Cholesterin, Triglyceride, Blutzucker, maximale Leistung (Watt), maximale Herzfrequenz (Puls/min), Leistung pro kg/Körpergewicht, individuelle anaerobe Schwellenleistung (IANS in Watt), individuelle anaerobe Schwellen-Herzfrequenz (IANSHF in Puls/min), individuelle anearobe Schwellenleistung pro kg Körpergewicht (IANSL/kg in Watt). Die leistungsdiagnostischen Parameter wurden am Ergometer bestimmt. Die Ermittlung der Schwellenleistungen wurden nach dem Conconi-Test (Boutellier, U.: Physiological basis for the measurement of aerobic capacity, Schweiz Rundsch. Med. Prax. (1989), 78(35): 921-4; Conconi F., et al.: Determination of the anaerobic threshold by a noninvasive field test in runners, J. Appl. Physiol. (1982), 52(4): 869-73) vorgenommen.

### Ergebnisse:

Die Trendstudie wurde von 29 Personen beendet. 11 Teilnehmer mussten wegen mangelnder Compliance ausgeschlossen werden.

Die Auswertung der einzelnen Parameter ergab nach 10-wöchigem Prüfzeitraum folgende Abweichungen in %:

**Tabelle 3:**

| **Veränderung der leistungsdiagnostischen Parameter** | | |
|---|---|---|
| Leistungskriterium | Veränderung der Leistungskriterien in % | |
| | Verum-Gruppe (n=12) | Placebo-Gruppe (n=17) |
| Maximale Leistung (Watt) | 13,75 | 13,24 |
| Maximale Herzfrequenz (Puls/min) | -6,17 | -1,94 |
| Maximale Leistung/kg KG(Watt/kg) | 0,18 | 0,15 |
| IANSL (Watt) | 13,89 | 6,39 |
| IANSHF (Puls/min) | -3,25 | 1,75 |
| IANSL/kg (Watt/kg) | 0,18 | 0,08 |

**Tabelle 4:**

| Laborwert | Veränderung in % (mg/100 ml) | |
|---|---|---|
| | Verum-Gruppe (n = 8) | Placebo-Gruppe (n = 5) |
| Gesamtcholesterin | -9,1 % (von 221,4 auf 201,2) | -8,15 % (von 214,2 auf 196,8) |
| HDL-Cholesterin | +61,9 % (von 43,3 auf 60) | +45,5 % (von 50,3 auf 73,4) |

Unter identischen Trainingsbedingungen konnte in der Verum-Gruppe eine deutliche Erhöhung der individuellen anaeroben Schwellenleistung (IANS in Watt) festgestellt werden. Das entspricht einer Rechtsverschiebung der Lactatkurve. Alkalisalze (z.B. Natriumbicarbonat; Hydrogencarbonat) sind an der Regulation des Säure-Basen-Haushaltes beteiligt und dienen u.a. zur Neutralisation von im Zuge der anaeroben Energiebereitstellung anfallenden Lactats. Ein hoher Gehalt an basischen Nährstoffen kann die Aufnahme von Lactat aus der Muskelzelle erhöhen, d.h. das Absinken des pH-Wertes in der Muskelzelle minimieren, und so Ermüdungserscheinungen entgegenwirken.

## Patentansprüche

1. Verwendung von Lösungen von Salzen organischer Säuren, ausgewählt aus Hydrogencarbonaten oder Carbonaten, zur Beschleunigung des Lactatabbaues.

2. Verwendung von Lösungen von Salzen organischer Säuren, ausgewählt aus Hydrogencarbonaten oder Carbonaten, zur Erhöhung der anaeroben Schwellenleistung.

3. Verwendung nach Anspruch 1 oder 2, **dadurch gekennzeichnet, dass** weiters Salze anderer organischer Säuren, ausgewählt aus einem oder mehreren Salzen von organischen Säuren mit einem C₁-C₆-Grundkörper, verwendet werden.

4. Verwendung nach Anspruch 3, **dadurch gekennzeichnet, dass** die Salze anderer organischer Säuren ausgewählt sind aus Citraten, Hydrogencitraten, Acetaten, Gluconaten, Tartraten, Salze anderer C₂-C₆-Säuren oder Mischungen dieser Salze.

5. Verwendung nach einem der Ansprüche 1 bis 4, **dadurch gekennzeichnet, dass** die Salze organischer Säuren Na-, K-, NH₄-, Ca- oder Mg-Salze sind.

6. Wässerige basenhaltige Mikronährstoffmischung, enthaltend Salze organischer Säuren, ausgewählt aus Hydrogencarbonaten oder Carbonaten, Vitamine des B-Komplexes, Vitamin C, Eisen, Chrom, Selen, Zink, Mangan und Kupfer, wobei die Mikronährstoffmischung einen osmotischen Druck von 650 kPa oder weniger aufweist.

7. Zusammensetzung nach Anspruch 6, **dadurch gekennzeichnet, dass** sie in wässeriger Lösung einen pH von 7,5 oder höher aufweist.

8. Zusammensetzung nach Anspruch 6 oder 7, **dadurch gekennzeichnet, dass** sie in wässeriger Lösung einen pH von 8,0 oder höher, insbesondere von 8,5 oder höher, aufweist.

9. Zusammensetzung nach einem der Ansprüche 6 bis 8, **dadurch gekennzeichnet, dass** sie weiters Vitamin E, Provitamin A, Molybdän, Magnesium, Chlorid, Natrium, Calcium, Kalium, Phosphat oder Mischungen davon enthält.

10. Zusammensetzung nach einem der Ansprüche 6 bis 9, **dadurch gekennzeichnet, dass** sie Kohlenhydrate in einer Menge von unter 30%, insbesondere von unter 20%, bezogen auf das Gesamtgewicht der trockenen Zusammensetzung enthält.

11. Zusammensetzung nach einem der Ansprüche 6 bis 10, **dadurch gekennzeichnet, dass** sie im Wesentlichen frei an Fetten ist.

12. Zusammensetzung nach einem der Ansprüche 6 bis 11, **dadurch gekennzeichnet, dass** sie im Wesentlichen frei an Proteinen ist.

13. Zusammensetzung nach einem der Ansprüche 6 bis 12, **dadurch gekennzeichnet, dass** die Vitamine des B-Komplexes ausgewählt sind aus Vitamin B1, B2, B6, B12, Biotin, Folsäure, Pantothensäure, Niacin und Mischungen davon.

14. Zusammensetzung nach einem der Ansprüche 6 bis 13, **dadurch gekennzeichnet, dass** sie unabhängig voneinander Salze organischer Säuren, ausgewählt aus Hydrogencarbonaten oder Carbonaten, in einer Menge von 0,2 bis 20%, vorzugsweise von 1 bis 10%, insbesondere von 2 bis 7%, Vitamine des B-Komplexes in einer Menge von 0,0001 bis 2%, vorzugsweise von 0,001 bis 1%, insbesondere von 0,01 bis 0,5%, Vitamin C in einer Menge von 0,001 bis 5%, vorzugsweise von 0,01 bis 2%, insbesondere von 0,1 bis 1%, Eisen in einer Menge von 0,0001 bis 0,5%, vorzugsweise von 0,001 bis 0,2%, insbesondere von 0,01 bis 0,1%, Chrom in einer Menge von 0,000001 bis 0,01%, vorzugsweise von 0,00001 bis 0,001%, insbesondere von 0,0001 bis 0,001%, Selen in einer Menge von 0,000001 bis 0,01%, vorzugsweise von 0,00001 bis 0,001%, insbesondere von 0,0001 bis 0,001%, Zink in einer Menge von 0,0001 bis 0,5%, vorzugsweise von 0,001 bis 0,2%, insbesondere von 0,01 bis 0,1%, Mangan in einer Menge von 0,00001 bis 0,1%, vorzugsweise von 0,0001 bis 0,01%, insbesondere von 0,001 bis 0,01% und Kupfer in einer Menge von 0,00001 bis 0,1%, vorzugsweise von 0,0001 bis 0,01%, insbesondere von 0,001 bis 0,01%, jeweils bezogen auf das Gesamtgewicht der trockenen Zusammensetzung enthält.

15. Zusammensetzung nach einem der Ansprüche 6 bis 14, **dadurch gekennzeichnet, dass** sie weiters Gemüseextrakte, insbesondere Karottenpulver, Fruchtpulver, insbesondere Orangenpulver, organische Säuren, Farbstoffe, insbesondere beta-Carotin und Anthocyane, Süßungsmittel, insbesondere Aspartame, oder Mischungen davon umfasst.

16. Zusammensetzung nach Anspruch 15, **dadurch gekennzeichnet, dass** sie Zitronensäure als organische Säure umfasst.

17. Zusammensetzung nach einem der Ansprüche 6 bis 16, **dadurch gekennzeichnet, dass** sie in Dosisform vorliegt.

18. Zusammensetzung nach Anspruch 17, **dadurch gekennzeichnet, dass** sie in einer Tagesdosisform oder in einer Einzeldosisform vorliegt.

19. Zusammensetzung nach einem der Ansprüche 6 bis 18, **dadurch gekennzeichnet, dass** sie Cyanocobalamin, Natriumselenit, Natriummolybdat, Chrom-III-chlorid-hexahydrat, Riboflavin, Aneurin-HCl, Pyridoxol-HCl, Calciumpantothenat, dl-alpha-Tocopherol, Kupfergluconat, Mangangluconat, Zinkgluconat, Eisengluconat, Natriumascorbat oder Mischungen davon enthält.

20. Zusammensetzung nach einem der Ansprüche 6 bis 19, **dadurch gekennzeichnet, dass** sie Zitronensäure, Orangenfruchtpulver, Karottenextrakt, Calciumglycerophosphat, Magnesiumglycerophosphat, Natriumchlorid, Süßungsmittel, insbesondere Aspartam, oder Mischungen davon enthält.

21. Zusammensetzung nach einem der Ansprüche 6 bis 20, **dadurch gekennzeichnet, dass** sie als wässerige Lösung vorliegt in einer Konzentration von 0,5 bis 200 g, insbesondere 2 bis 70 g, insbesondere von 5 bis 20 g, pro 250 ml Wasser.

22. Zusammensetzung nach einem der Ansprüche 6 bis 21, **dadurch gekennzeichnet, dass** sie Natrium-, Ammonium-, Kaliumhydrogencarbonat oder Mischungen davon enthält.

23. Zusammensetzung nach einem der Ansprüche 6 bis 21, **dadurch gekennzeichnet, dass** sie Selen in Selenit- oder Selenat-Form enthält.

24. Zusammensetzung nach einem der Ansprüche 6 bis 23, **dadurch gekennzeichnet, dass** sie Metalle in Gluconat-Form enthält.

25. Zusammensetzung nach einem der Ansprüche 6 bis 24, **dadurch gekennzeichnet, dass** sie Natrium-, Kalium-, Calcium-, Magnesium-Carbonat, -Bicarbonat, Natrium-, Kalium-, Calcium-, Magnesium-Salze von organischen Säuren, insbesondere Citrat-, Mono- und Hydrogencitrat, Tartrat, Gluconat und andere organische Salze oder Mischungen davon enthält.

26. Verwendung einer Zusammensetzung nach einem der Ansprüche 6 bis 25 zur Beschleunigung des Lactatabbaues.

27. Verwendung einer Zusammensetzung nach einem der Ansprüche 6 bis 25 zur Verbesserung der Ausdauer bzw. Leistungsfähigkeit.

28. Verwendung einer Zusammensetzung nach einem der Ansprüche 6 bis 25 zur Erhöhung der anaeroben Schwellenleistung.

29. Verwendung einer Zusammensetzung nach einem der Ansprüche 6 bis 25 zur Verhinderung oder Verzögerung von Ermüdungserscheinungen.

30. Verwendung einer Zusammensetzung nach einem der Ansprüche 6 bis 25 als Basis für ein leistungssteigerndes und -verlängerndes Getränk für Sportler und Nicht-Sportler.

## Claims

1. The use of solutions of salts of organic acids, selected from hydrogen carbonates or carbonates, to accelerate lactate degradation.

2. The use of solutions of salts of organic acids, selected from hydrogen carbonates or carbonates, to increase the anaerobic threshold performance.

3. The use according to claim 1 or 2, **characterized in that** other organic acid salts, selected from one or more salts of organic acids having a C₁-C₆ base body, are used in addition.

4. The use according to claim 3, **characterized in that** the other organic acid salts are selected from citrates, hydrogen citrates, acetates, gluconates, tartrates, salts of other C₂-C₆ acids or mixtures of these salts.

5. The use according to any one of claims 1 to 4, **characterized in that** said organic acid salts are Na, K, NH₄, Ca or Mg salts.

6. An aqueous base-containing micronutrient mixture comprising organic acid salts selected from hydrogen carbonates or carbonates, B-complex vitamins, vitamin C, iron, chromium, selenium, zinc, manganese and copper, said micronutrient mixture having an osmotic pressure of 650 kPa or less.

7. A composition according to claim 6, **characterized in that** it has a pH of 7.5 or more in an aqueous solution.

8. A composition according to claim 6 or 7, **characterized in that** it has a pH of 8.0 or more, in particular 8.5 or more, in an aqueous solution.

9. A composition according to any one of claims 6 to 8, **characterized in that** it further comprises vitamin E, provitamin A, molybdenum, magnesium, chloride, sodium, calcium, potassium, phosphate or mixtures thereof.

10. A composition according to any one of claims 6 to 9, **characterized in that** it comprises carbohydrates in an amount of less than 30%, particularly less than 20%, based on the total weight of the dry composition.

11. A composition according to any one of claims 6 to 10, **characterized in that** it is substantially free of fats.

12. A composition according to any one of claims 6 to 11, **characterized in that** it is substantially free of proteins.

13. A composition according to any one of claims 6 to 12, **characterized in that** the B-complex vitamins are selected from vitamins B1, B2, B6, B12, biotin, folic acid, pantothenic acid, niacin and mixtures thereof.

14. A composition according to any one of claims 6 to 13, **characterized in that** it independently comprises salts of organic acids, selected from hydrogen carbonates or carbonates, in amounts of from 0.2 to 20%, preferably 1 to 10%, in particular 2 to 7%; B-complex vitamins in amounts of from 0.0001 to 2%, preferably 0.001 to 1%, in particular 0.01 to 0.5%; vitamin C in an amount of from 0.001 to 5%, preferably 0.01 to 2%, in particular 0.1 to 1%; iron in an amount of from 0.0001 to 0.5%, preferably 0.001 to 0.2%, in particular 0.01 to 0.1%; chromium in an amount of from 0.000001 to 0.01%, preferably 0.00001 to 0.001%, in particular 0.0001 to 0.001%; selenium in an amount of from 0.000001 to 0.01%, preferably 0.00001 to 0.001%, in particular 0.0001 to 0.001%; zinc in an amount of from 0.0001 to 0.5%, preferably 0.001 to 0.2%, in particular 0.01 to 0.1%; manganese in an amount of from 0.00001 to 0.1%, preferably 0.0001 to 0.01%, in particular 0.001 to 0.01%; and copper in an amount of from 0.00001 to 0.1%, preferably 0.0001 to 0.01%, in particular 0.001 to 0.01%; each based on the total weight of the dry composition.

15. A composition according to any one of claims 6 to 14, **characterized in that** it further comprises vegetable extracts, in particular carrot powder, fruit powder, in particular orange powder, organic acids, coloring agents, in particular beta-carotene and anthocyans, sweetening agents, in particular aspartames, or mixtures thereof.

16. A composition according to claim 15, **characterized in that** it comprises citric acid as an organic acid.

17. A composition according to any one of claims 6 to 16, **characterized in that** it is present in dose form.

18. A composition according to claim 17, **characterized in that** it is present in a daily dose form or a single dose form.

19. A composition according to any one of claims 6 to 18, **characterized in that** it comprises cyanocobalamine, sodium selenite, sodium molybdate, chromium-III-chloride hexahydrate, riboflavin, aneurin-HCl, pyridoxine-HCl, calcium pantothenate, dl-alpha-to-copherol, copper gluconate, manganese gluconate, zinc gluconate, iron gluconate, sodium ascorbate or mixtures thereof.

20. A composition according to any one of claims 6 to 19, **characterized in that** it comprises citric acid, orange fruit powder, carrot extract, calcium glycerophosphate, magnesium glycerophosphate, sodium chloride, sweetening agents, in particular aspartame, or mixtures thereof.

21. A composition according to any one of claims 6 to 20, **characterized in that** it is present as an aqueous solution at a concentration of from 0.5 to 200 g, in particular 2 to 70 g, in particular 5 to 20 g, per 250 ml water.

22. A composition according to any one of claims 6 to 21, **characterized in that** it comprises sodium, ammonium, potassium hydrogen carbonates or mixtures thereof.

23. A composition according to any one of claims 6 to 21, **characterized in that** it comprises selenium in selenite or selenate form.

24. A composition according to any one of claims 6 to 23, **characterized in that** it comprises metals in gluconate form.

25. A composition according to any one of claims 6 to 24, **characterized in that** it comprises sodium, potassium, calcium, magnesium carbonates or bicarbonates, sodium, potassium, calcium, magnesium salts of organic acids, in particular citrates, mono- and hydrogen citrates, tartrate, gluconate and other organic salts or mixtures thereof.

26. The use of a composition according to any one of claims 6 to 25 to accelerate lactate degradation.

27. The use of a composition according to any one of claims 6 to 25 to improve endurance and performance.

28. The use of a composition according to any one of claims 6 to 25 to increase the anaerobic threshold performance.

29. The use of a composition according to any one of claims 6 to 25 to prevent or slow down symptoms of fatigue.

30. The use of a composition according to any one of claims 6 to 25 as a basis for a performance-increasing and -prolonging drink for athletes and non-athletes.

## Revendications

1. Utilisation de solutions de sels d'acides organiques, choisis parmi les hydrogénocarbonates ou les carbonates, pour accélérer la dégradation de lactate.

2. Utilisation de solutions de sels d'acides organiques, choisis parmi les hydrogénocarbonates ou les carbonates, pour augmenter la production-seuil anaérobie.

3. Utilisation selon la revendication 1 ou 2, **caractérisée en ce que** d'autres sels d'autres acides organiques, choisis parmi un ou plusieurs sels d'acides organiques avec un corps de base en C₁-C₆, sont utilisés.

4. Utilisation selon la revendication 3, **caractérisée en ce que** les sels d'autres acides organiques sont choisis parmi les citrates, les hydrogénocitrates, les acétates, les gluconates, les tartrates, les sels d'autres acides en C₂-C₆ ou des mélanges de ces sels.

5. Utilisation selon l'une quelconque des revendications 1 à 4, **caractérisée en ce que** les sels d'acides organiques sont des sels de Na, de K, de NH₄, de Ca ou de Mg.

6. Mélange aqueux de substances micronutritives contenant une base, contenant des sels d'acides organiques, choisis parmi des hydrogénocarbonates ou des carbonates, des vitamines du complexe B, la vitamine C, du fer, du chrome, du sélénium, du zinc, du manganèse et du cuivre, où le mélange de substances micronutritives présente une pression osmotique de 650 kPa ou moins.

7. Composition selon la revendication 6, **caractérisée en ce qu'**elle présente en solution aqueuse, un pH de 7,5 ou plus.

8. Composition selon la revendication 6 ou 7, **caractérisée en ce qu'**elle présente en solution aqueuse, un pH de 8,0 ou plus, en particulier de 8,5 ou plus.

9. Composition selon l'une quelconque des revendications 6 à 8, **caractérisée en ce qu'**elle contient en outre, la vitamine E, la provitamine A, le molybdène, le magnésium, un chlorure, le sodium, le calcium, le potassium, un phosphate ou des mélanges de ceux-ci.

10. Composition selon l'une quelconque des revendications 6 à 9, **caractérisée en ce qu'**elle contient des hydrates de carbone en une quantité de moins de 30%, en particulier de moins de 20%, sur base du poids total de la composition sèche.

11. Composition selon l'une quelconque des revendications 6 à 10, **caractérisée en ce qu'**elle est essentiellement exempte de graisses.

12. Composition selon l'une quelconque des revendications 6 à 11, **caractérisée en ce qu'**elle est essentiellement exempte de protéines.

13. Composition selon l'une quelconque des revendications 6 à 12, **caractérisée en ce que** les vitamines du complexe B sont choisies parmi les vitamines B1, B2, B6, B12, la biotine, l'acide folique, l'acide pantothénique, la niacine et leurs mélanges.

14. Composition selon l'une quelconque des revendications 6 à 13, **caractérisée en ce qu'**elle contient indépendamment l'un de l'autre, des sels d'acides organiques, choisis parmi les hydrogénocarbonates et les carbonates, en une quantité de 0,2 à 20%, de préférence de 1 à 10%, en particulier de 2 à 7%, les vitamines du complexe B en une quantité de 0,0001 à 2%, de préférence de 0,001 à 2%, en particulier de 0,01 à 0,5%, la vitamine C en une quantité de 0,001 à 5%, de préférence de 0,01 à 2%, en particulier de 0,1 à 1%, le.fer en une quantité de 0,0001 à 0,5%, de préférence de 0,001 à 0,2%, en particulier de 0,01 à 0,1%, le chrome en une quantité de 0,000001 à 0,01%, de préférence de 0,00001 à 0,001%, en particulier de 0,0001 à 0,001%, le sélénium en une quantité de 0,000001 à 0,01%, de préférence de 0,00001 à 0,001%, en particulier de 0,0001 à 0,001%, le zinc en une quantité de 0,0001 à 0,5%, de préférence de 0,001 à 0,2%, en particulier de 0,01 à 0,1 %, le manganèse en une quantité de 0,00001 à 0,1%, de préférence de 0,0001 à 0,01%, en particulier de 0,001 à 0,01%,, et le cuivre en une quantité de 0,00001 à 0,1%, de préférence de 0,0001 à 0,01%, en particulier de 0,001 à 0,01%, chaque fois sur base du poids total de la composition sèche.

15. Composition selon l'une quelconque des revendications 6 à 14, **caractérisée en ce qu'**elle comprend d'autre part, des extraits de légumes, en particulier de la poudre de carotte, de la poudre de fruit, en particulier de la poudre d'orange, des acides organiques, des colorants, en particulier le bêta-carotène et des anthocyanes, des édulcorants, en particulier l'aspartame, ou leurs mélanges.

16. Composition selon la revendication 15, **caractérisée en ce qu'**elle comprend l'acide citrique comme acide organique.

17. Composition selon l'une quelconque des revendications 6 à 16, **caractérisée en ce qu'**elle se présente sous forme de dosage.

18. Composition selon la revendication 17, **caractérisée en ce qu'**elle se présente sous une forme de dosage quotidien ou une forme de dosage unique.

19. Composition selon l'une quelconque des revendications 6 à 18, **caractérisée en ce qu'**elle contient la cyanocobalamine, le sélénite de sodium, le molybdate de sodium, le chlorure de chrome III hexahydraté, la riboflavine, l'aneurine.HCl, le pyridoxol.HCl, le pantothénate de calcium, le dl-alpha-tocophérol, le gluconate de cuivre, le gluconate de manganèse, le gluconate de zinc, le gluconate de fer, l'ascorbate de sodium ou leurs mélanges.

20. Composition selon l'une quelconque des revendications 6 à 19, **caractérisée en ce qu'**elle contient l'acide citrique, de la poudre d'orange, un extrait de carotte, le glycérophosphate de calcium, le glycérophosphate de magnésium, le chlorure de sodium, un édulcorant, en particulier l'aspartame ou leurs mélanges.

21. Composition selon l'une quelconque des revendications 6 à 20, **caractérisée en ce qu'**elle se présente sous la forme d'une solution aqueuse, en une concentration de 0,5 à 200 g, en particulier de 2 à 70 g, en particulier de 5 à 20 g, par 250 ml d'eau.

22. Composition selon l'une quelconque des revendications 6 à 21, **caractérisée en ce qu'**elle contient l'hydrogénocarbonate de sodium, d'ammonium, de potassium ou leurs mélanges.

23. Composition selon l'une quelconque des revendications 6 à 21, **caractérisée en ce qu'**elle contient le sélénium sous la forme de sélénite ou de sélénate.

24. Composition selon l'une quelconque des revendications 6 à 23, **caractérisée en ce qu'**elle contient les métaux sous la forme de gluconate.

25. Composition selon l'une quelconque des revendications 6 à 24, **caractérisée en ce qu'**elle contient le carbonate ou bicarbonate de sodium, de potassium, de calcium, de magnésium, des sels de sodium, de potassium, de calcium, de magnésium d'acides organiques, en particulier le citrate, le mono- et l'hydrogénocitrate, le tartrate, le gluconate ou d'autres sels organiques ou leurs mélanges.

26. Utilisation d'une composition selon l'une des revendications 6 à 25, pour accélérer la dégradation de lactate.

27. Utilisation d'une composition selon l'une des revendications 6 à 25, pour améliorer l'endurance et respectivement, la capacité de performance.

28. Utilisation d'une composition selon l'une des revendications 6 à 25, pour augmenter la production-seuil anaérobie.

29. Utilisation d'une composition selon l'une des revendications 6 à 25, pour empêcher ou retarder les phénomènes de fatigue.

30. Utilisation d'une composition selon l'une des revendications 6 à 25, comme base pour une boisson augmentant ou allongeant la performance pour sportifs ou non sportifs.
